# EUROPEAN PATENT SPECIFICATION

(11) **EP 1 342 470 B1**
(45) Date of publication and mention of the grant of the patent: **15.08.2007**
(21) Application number: 02386005.9
(22) Date of filing: 04.03.2002
(51) Int. Cl.: A61K 9/20, A61K 9/28, A61K 31/545

(54) **Fast release cefuroxime axetil compositions**
Zusammensetzungen mit schneller Freisetzung enthaltend Cefuroxime Axetil
Compositions à libération rapide de Cefuroxime Axetil

(43) Date of publication of application: 10.09.2003
(73) Proprietor: Orbus Pharma Inc., Markham, ON L3R 8T4 (CA)
(72) Inventor: Karavas, Evangelos, Agios Dimitrios, Attikis 17342 (GR); Kotzagiorgis, Evangelos, Halandri, Attikis 15234 (GR)
(74) Representative: Vogel, Andreas

(56) References cited:
- WO-A-99/62559
- US-A- 4 602 012

## Description

This innovation concerns a formulation of a fast release dosage form of Cefuroxime Axetil, which provides optimized release of the drug according to the dissolution profile limits of the USP

In treatment of the respiratory tract infections fast release dosage formulations are particularly necessary in order to achieve high plasma therapeutic levels in as short a time as possible.

Specifically, the innovation constitutes a fast release pharmaceutical composition comprising an effective amount of Cefuroxime Axetil, as an active ingredient, an alkaline carbonate, a polycarboxylic acid as a. buffering agent, and at least one excipient, wherein said composition is in a tablet form and comprises from 20 % to 90 % by weight of said Cefuroxime Axetil and from 0,1 % to 10 % by weight of said alkaline carbonate.

### Background of the invention:

Cefuroxime Axetil, the 1-acetoxyethyl ester of Cefuroxime (1-acetoxyethyl-(6R, 7R)-3-carbamoyloxymethyl-7-[(Z)-2-(fur-2-yl)-2-methoxyiminoacetamido]ceph-3-em-4-carboxylate), is the oraly bioavailable form of the broad spectrum antibiotic Cefuroxime.

Prior art reveals substancial difficulties in producing oral solid formulations of Cefuroxime Axetil providing high bioavailability. Amorphous Cefuroxime Axetil, which has been disclosed in patent EP 0107256, is more soluble in aqueous media than the crystalline form, but shows a strong tendency to gel formation, leading to low dissolution profiles. Several innovations in order to overcome this problem include controlling of the film coat rupture time and using a tablet core rich in a super disintegrant (EP 0223365), converting Cefuroxime Axetil to a "non-gelatinous" form of solid solution or dispersion (WO 0030647), or to a coprecipitate with a water-soluble excipient (WO 9908683) etc.

WO-A-9962559 discloses a tablet made by compression of granules comprising Cefuroxime Axetil and sorbitol mixed with crospovidone, sodium carbonate, magnesium stearate and colloidal silicon dioxide.

As mentioned above the critical factor for the bioavailability of the active material, Cefuroxime Axetil tablet compositions is the disintegration of tablet core leading to a poor dissolution of drug in the medium.

The reasons for this are the physical properties of Cefuroxime Axetil. The drug forms a gelatinous mass at 37°C, which does not allow aqueous media to penetrate into the tablet matrix. This mechanism leads to a long disintegration time and thus, to poor dissolution, and poor absorption of Cefuroxime Axetil by gasrtointestinal tract. Moreover, coatings applied on tablet core, in order to mask bitter taste of drug, intensify slow release of Cefuroxime Axetil as well.

The EP 0223365 describes a method of preparation of film coated tablets, in order to avoid slow release profiles of the drug. According to this patent, a super disintegrant (cross-carmellose sodium) is used in the matrix, whilst the tablets are coated with a specific polymer (hydroxypropylmethylcellulose 5 cps or 6 cps) which is ruptured in less than 40 seconds from tablet core.

The release profile of formulation in the EP 0223365 is according to dissolution profile limits mentioned in USP for Cefuroxime Axetil tablets.

### Detailed description of the invention

The present invention, concerning a fast release formulation containing Cefuroxime Axetil as the active drug component, was focused on:
1) Preparation of a matrix with an optimum disintegration time, avoiding the formation of the gelatinous layer.
2) Preparation of a coating based on soluble polymers and surfactants in order to improve water penetration, and being capable to mask the bitter taste of Cefuroxime Axetil.

More details are described below:

### 1) CORE - FORMULATIONS

In the course of this investigation it was found that partially water-soluble disintegrants (e.g. starch 1500, Primogel etc.) lead directly to the formation of a gelatinous mass, thus intensifying disintegration times to more than 30 min. and resulting in slow dissolution profiles.

The use of extra quantities of a surfactant such as Sodium Laurul Sulphate (SLS) did not improve the disintegration of prepared tablets. The disintegration time was more than 12 min.

The combination of disintegrants partially soluble and insoluble in water (Primogel - Crospovidone, 1:1) improved disintegration time (7 min.), but gel formation still occurred.

The use of water insoluble Crospovidone as a disintegrant in combination with a sugar alcohol(e.g. mannitol, sorbitol etc) as a filler reduced disintegration time to 5 minutes.

All of the above compositions contained Aerosil, Sodium Lauryl Sulphate and Magnesium Stearate as lubricants. However, Magnesium Stearate was found to have a negative effect on the disintegration and dissolution properties due to its hydrophobic character. For this reason it was avoided from the remainder of the trials and was replaced by Talc.

Although the disintegration time was satisfactory, the dissolution profile was not acceptable because a gel layer continued to form, preventing water penetration throughout the whole mass of the matrix. Therefore the quantity of the disintegrant in the center of the matrix was not working, as insufficient contact with the water was occurring, in order for it to swell and consequently act in the desired manner.

The formation of channels throught the matrix, following contact of the matrix with water is necessary for water penetration throughout the whole mass of the matrix. This mechanism leads to rapid action of the disintegrant and rapid disintegration time.

For the formation of these channels, alkaline carbonate salts (e.g. sodium bicarbonate, potassium bicarbonate) have been used in quantities from 0,1% to 10 %. Their effect is based on the following indicative reaction:

NaHC03 + HCl^{→} Na Cl + H 2 O + CO2 ↑

The produced CO2 is the factor, which forms the above-mentioned channels. The use of the surfactant Sodium Lauryl Sulphate improves water penetration by reducing surface tensions.

Since Cefuroxime axetil is known to be sensitive in alkaline conditions, a polycarboxylic acid such as citric or tartaric acid was also incorporated as a buffering agent.

### 2) COATING

The EP 0223365 claims the use of a coating material, specifically Hydoxypropylmethylellulose 5 or 6 . Apparently the patent uses the term "rupture" because the mentioned polymers are slowly dispersed in water, giving colloidal dispersions so the polymer is "ruptured" before their solubilization.

In order to obtain more rapid disintegration of tablets, several other water-soluble coating polymers have been tested instead of Hydroxypropylmethylcellulose, such as PEG, PVA, PVP. These soluble polymers in combination with a surfactant have been used, in order to improve water penetration.

Specifically a coating based on soluble polymers such as Polyvinylpyrrolidone (PVP K 30) has been developed in combination with a surfactant such as Sodium Docusate. The surfactants work in two ways:
- Lead to a rapid dissolution (not "rupture") of the coating (less than 10 sec).
- Improve water penetration directly to the mass of the core. In this way the problem of the formation of the gelatinous mass, which is observed in conventional coatings, is completely avoided.

The dissolution time of the films prepared as above is less than 10 seconds.

### IN VITRO TESTS-Dissolution of the coated tablets

The dissolution characteristics of the coated tablets prepared according to the present invention, as described above and further exemplified hereafter in the examples, were tested according to the method set forth in the monograph of Cefuroxime Axetil tablets in USP 23. Average results of dissolution tests of the coated tabled prepared according to the present invention are the following.

| | | |
|---|---|---|
| Dissolution According to USPXXIII: | 75 % in 15 min | (USP Limit : at least 60%) |
| | 85 % in 30 min | |
| | 91 % in 45 min | (USP Limit: at least 75 %) |

The other tests (Assay, Impurities, Friability, Compressibility, Stability e.t.c.) are acceptable.

### Example 1 (Mannitol Formula)

| CEFUROXIME 500 mg/TAB | |
|---|---|
| Cefuroxime Axetil | 602.0 mg |
| Mannitol | 76.0 mg |
| Citric acid | 20.0 mg |
| Crospovidone | 140.0 mg |
| Talc | 24.0 mg |
| Sodium Lauryl Sulphate | 20.0 mg |
| Sodium Bicarbonate | 20.0 mg |
| Aerosil | 2.0 mg |
| **Total weight** | **904.0 mg** |

The other strengths are linear to the above.

### Manufacturing process is described below.

This formulation gave the following results:

| | |
|---|---|
| Hardness | 112 Nt |
| Disintegration (uncoated tablets) | 40 sec |

### COATING

| | |
|---|---|
| PVP | 24 mg |
| Sodium Docusate | 1.2 mg |
| Propylene Glycol | 0.021 mg |
| Titanium Dioxide | 6.4 mg |
| Solvent | Ethanol |
| **Total coating weight per 500 mg tablet** | **31.62 mg** |

### COATING DISSOLUTION <10 sec

### TOTAL TABLET WEIGHT 935.62 mg

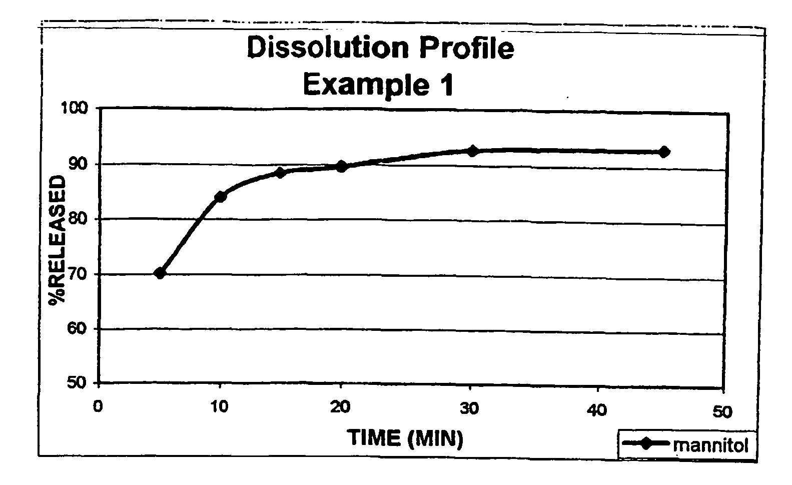

| TIME(min) | % RELEASED |
|---|---|
| 5 | 70,13 |
| 10 | 84,06 |
| 15 | 88,57 |
| 20 | 89,64 |
| 30 | 92,8 |
| 45 | 93,17 |

### Example 2 (Sorbitol Formula)

| CEFUROXIME 500 mg/AB | |
|---|---|
| Cefuroxime Axetil | 602.0 mg |
| Sorbitol | 76.0 mg |
| Tartaric acid | 20 mg |
| Crospovidone | 140.0 mg |
| Talc | 24.0 mg |
| Sodium Lauryl Sulphate | 20.0 mg |
| Sodium Bicarbonate | 20.0 mg |
| Aerosil | 2.0 mg |
| **Total weight** | **904.0 mg** |

The other strengths are linear to the above

### Manufacturing process as described below.

This formulation gave the following results:

| | |
|---|---|
| Hardness | 128 Nt |
| Disintegration (uncoated tablets) | 42 sec |

### COATING

| | |
|---|---|
| PVP | 24 mg |
| Sodium Docusate | 1.2 mg |
| Propylene Glycol | 0.021 mg |
| Titanium Dioxide | 6.4 mg |
| Solvent | Ethanol |
| Total coating weight per 500 mg tablet | 31.62 mg |

### COATING DISSOLUTION <10 sec

### TOTAL TABLET WEIGHT 935.62 mg

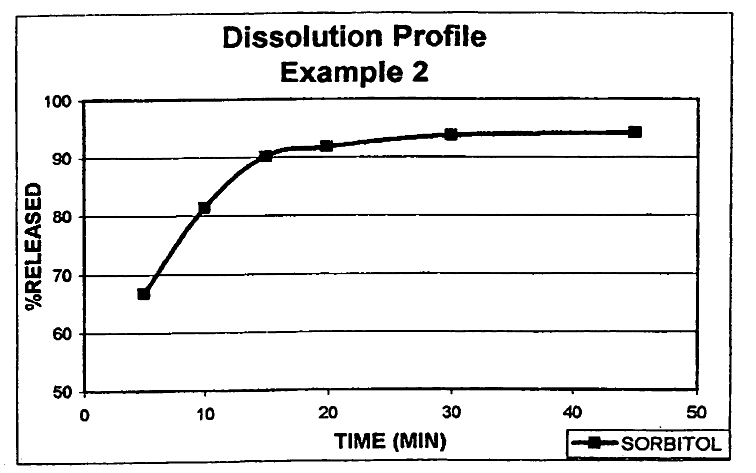

| TIME(min) | % RELEASED |
|---|---|
| 5 | 66,8 |
| 10 | 81,4 |
| 15 | 90,2 |
| 20 | 91,8 |
| 30 | 93,8 |
| 45 | 94,2 |

### MANUFACTURING PROCESS

• **Weighing**

| | |
|---|---|
| Cefuroxime Axetil | 602 mg |
| Mannitol or Sorbitol | 76 mg |
| Citric or Tartaric acid | 20 mg |
| Crospovidone | 100 mg |
| Talc | 12 mg |
| Sodium Bicarbonate | 12 mg |
| Sodium Lauryl Sulphate | 10 mg. |

• Sieving and Mixing
• Slugging
• Granulation

This product is the INTERNAL phase
• Weighing

| | |
|---|---|
| Crospovidone | 40 mg |
| Talc | 12 mg |
| NaHCO₃ | 8 mg |
| Sodium Lauryl Sulphate | 10 mg |
| Aerosil | 2 mg |

• Sieving and Mixing
• This product is the EXTERNAL phase
• Mixing of INTERNAL AND EXTERNAL PHASE
• Tableting
• Coating

In the following examples several coating materials based on soluble polymers in combination with a surfactant were applied on the cores described above in the examples 1 and 2.

### Example 3 (PVA coating)

The cores were produced according to Example 1

| | |
|---|---|
| **PVA** | **24 mg** |
| Sodium Docusate | 1.2 mg |
| Propylene Glycol | 0.021 mg |
| Titanium Dioxide | 6.4 mg |
| Solvent | Ethanol |
| **Total coating weight per 500 mg tablet** | **31.62 mg** |

COATING DISSOLUTION <10 sec

### Example 4 (PEG coating)

The cores were produced according to Example 1

| | |
|---|---|
| PEG | 24 mg |
| Sodium Docusate | 1.2 mg |
| Propylene Glycol | 0.021 mg |
| Titanium Dioxide | 6.4 mg |
| Solvent | Ethanol |
| **Total coating weight per 500 mg tablet** | **31.62 mg** |

COATING DISSOLUTION <10 sec

### Example 5 (TWEEN 80 coating)

The cores were produced according to Example 1

| | |
|---|---|
| PVP | 24 mg |
| TWEEN 80 | 1.2 mg |
| Propylene Glycol | 0.021 mg |
| Titanium Dioxide | 6.4 mg |
| Solvent | Ethanol |
| **Total coating weight per 500 mg tablet** | **31.62 mg** |

COATING DISSOLUTION <10 sec

### Example 6 (Poloxamer coating)

The cores were produced according to Example 1

| | |
|---|---|
| PVP | 24 mg |
| Poloxamer (F 68) | 1.2 mg |
| Propylene Glycol | 0.021 mg |
| Titanium Dioxide | 6.4 mg |
| Solvent | Ethanol |
| **Total coating weight per 500 mg tablet** | **31.62 mg** |

COATING DISSOLUTION <10 sec

## Claims

1. A fast release pharmaceutical composition comprising an effective amount of Cefuroxime Axetil, as an active ingredient, an alkaline carbonate, a polycarboxylic acid as a buffering agent, and at least one excipient, wherein said composition is in a tablet form and comprises from 20 % to 90 % by weight of said Cefuroxime Axetil and from 0,1 % to 10 % by weight of said alkaline carbonate.

2. The fast release pharmaceutical composition according to claim 1 , wherein it further comprises a disintegrants.

3. The fast release pharmaceutical composition according to claim 2, wherein said disintegrant is selected from the group of Polyvinylpyrrolidone, Sodium Carboxymethylcellulose; and Sodium Carboxymethyl Starch.

4. The fast release pharmaceutical composition according to claim 2, wherein said disintegrant is water-insoluble such as Crospovidone.

5. The fast release pharmaceutical composition according to claim 2, wherein it further comprises a sugar alcohol was a filler, such as Mannitol or Sorbitol.

6. The fast release pharmaceutical composition according to claim 1, wherein said alkaline carbonate is selected from the group consisting of Sodium Bicarbonate, and Potassium Bicarbonate.

7. The fast release pharmaceutical composition according to claim 1, wherein said polycarboxylic acid is selected from the group of citric acid or tartaric acid.

8. The fast release pharmaceutical composition according to any preceding claim, wherein said at least one excipient is selected from the group consisting of surfactants, and lubricants.

9. The fast release pharmaceutical composition according to claim 7, wherein said surfactant is selected from the group of Sodium Lauryl Sulphate, and Sodium Docusate.

10. The fast release pharmaceutical composition according to any preceding claim, wherein it comprises from 20% to 90% by weight of said Cefuroxime Axetil, from 0,1% to 10% by weight of said alkaline carbonate, from 5% to 40%, by weight of said sugar alcohol, from 3% to 50% by weight of said disintegrant, from 1% to 30% by weight of sail polycarboxylic acid and from 1% to 10% by weight of said surfactant.

11. The fast release pharmaceutical composition according to any preceding claim, wherein it comprises from 55% two 75% by weight of said Cefuroxime Axetil, from 8% to 15% by weight of said sugar alcohol, from 10% to 20% by weight of said disintegrant, from 1% to 5% by weigh alkaline carbonate and from 1%, to 5% by weight of said surfactant.

12. The fast release pharmaceutical composition according to any preceding claim, wherein said composition is in a tablet form and it further comprises a film coat which serves to improve the water penetration and to mask the bitter tastes of said Cefuroxime Axetil.

13. The fast release pharmaceutical composition according to claim 12, wherein said film coating being from 1% to 5% by weight of said total pharmaceutical compositions.

14. The fast release pharmaceutical composition according to claim 12, wherein said film coating comprises from 20% to 99% by weight of a soluble polymer such as PVA, and PEG, and from 1% to 20% by weight of a surfactant such as Sodium Docusate.

15. The fast release pharmaceutical composition according to claim 12 wherein said film coating comprises a water soluble polymer such as Polyvinylpyrrolidone, Polyethylenglycol, and Polyvinylalcohol.

16. The fast release pharmaceutical composition according to claim 12, wherein said film coating comprises a water soluble polymer in combination with a surfactant such as Sodium docusate, or Tween.

## Patentansprüche

1. Pharmazeutische Zusammensetzung zur schnellen Freisetzung, welche eine wirksame Menge an Cefuroxim-Axetil als aktiven Bestandteil sowie ein Alkalicarbonat, eine Polycarbonsäure als Puffersubstanz und mindestens einen Trägerstoff umfasst, wobei die Zusammensetzung in Tablettenform vorliegt und zwischen 20 Gewichts% und 90 Gewichts% an Cefuroxim-Axetil und zwischen 0,1 Gewichts% und 10 Gewichts% an Alkalicarbonat umfasst.

2. Pharmazeutische Zusammensetzung zur schnellen Freisetzung gemäß Anspruch 1, wobei diese darüber hinaus ein Sprengmittel umfasst.

3. Pharmazeutische Zusammensetzung zur schnellen Freisetzung gemäß Anspruch 2, wobei das Sprengmittel aus der Gruppe von Polyvinylpyrrolidon, Natriumcarboxymethylcellulose und Natriumcarboxymethylstärke gewählt ist.

4. Pharmazeutische Zusammensetzung zur schnellen Freisetzung gemäß Anspruch 2, wobei' das Sprengmittel wasserunlöslich ist wie etwa Crospovidon.

5. Pharmazeutische Zusammensetzung zur schnellen Freisetzung gemäß Anspruch 2, wobei diese darüber hinaus einen Zuckeralkohol wie etwa Mannit oder Sorbit als Füllstoff umfasst.

6. Pharmazeutische Zusammensetzung zur schnellen Freisetzung gemäß Anspruch 1, wobei das Alkalicarbonat aus der Gruppe, die aus Natriumhydrogencarbonat und Kaliumhydrogencarbonat besteht, gewählt ist.

7. Pharmazeutische Zusammensetzung zur schnellen Freisetzung gemäß Anspruch 1, wobei die Polycarbonsäure aus der Gruppe von Citronensäure oder Weinsäure gewählt ist.

8. Pharmazeutische Zusammensetzung zur schnellen Freisetzung gemäß einem beliebigen der vorhergehenden Ansprüche, wobei Trägerstoffe, von denen mindestens einer enthalten ist, aus der Gruppe gewählt sind, die aus Tensiden und Schmiermitteln besteht.

9. Pharmazeutische Zusammensetzung zur schnellen Freisetzung gemäß Anspruch 7, wobei das Tensid aus der Gruppe von Natriumlaurylsulfat und Natriumdioctylsulfosuccinat gewählt ist.

10. Pharmazeutische Zusammensetzung zur schnellen Freisetzung gemäß einem beliebigen der vorhergehenden Ansprüche, wobei diese zwischen 20 Gewichts% und 90 Gewichts% an Cefuroxim-Axetil, zwischen 0,1 Gewichts% und 10 Gewichts% an Alkalicarbonat, zwischen 5 Gewichts% und 40 Gewichts% an Zuckeralkohol, zwischen 3 Gewichts% und 50 Gewichts% an Sprengmittel, zwischen 1 Gewichts% und 30 Gewichts% an Polycarbonsäure und zwischen 1 Gewichts% und 10 Gewichts% an Tensid umfasst.

11. Pharmazeutische Zusammensetzung zur schnellen Freisetzung gemäß einem beliebigen der vorhergehenden Ansprüche, wobei diese zwischen 55 Gewichts% und 75 Gewichts% an Cefuroxim-Axetil, zwischen 8 Gewichts% und 15 Gewichts% an Zuckeralkohol, zwischen 10 Gewichts% und 20 Gewichts% an Sprengmittel, zwischen 1 Gewichts% und 5 Gewichts% an Alkalicarbonat und zwischen 1 Gewichts% und 5 Gewichts% an Tensid umfasst.

12. Pharmazeutische Zusammensetzung zur schnellen Freisetzung gemäß einem beliebigen der vorhergehenden Ansprüche, wobei die Zusammensetzung in Tablettenform vorliegt und darüber hinaus eine Filmbeschichtung umfasst, welche dazu dient, das Eindringverhalten von Wasser zu verbessern und den Bittergeschmack des Cefuroxim-Axetils zu maskieren.

13. Pharmazeutische Zusammensetzung zur schnellen Freisetzung gemäß Anspruch 12, wobei die Filmbeschichtung zwischen 1 Gewichts% und 5 Gewichts% der pharmazeutischen Zusammensetzung insgesamt ausmacht.

14. Pharmazeutische Zusammensetzung zur schnellen Freisetzung gemäß Anspruch 12, wobei die Filmbeschichtung zwischen 20 Gewichts% und 99% Gewichts% eines löslichen Polymers wie etwa PVA und PEG sowie zwischen 1 Gewichts% und 20% Gewichts% eines Tensids wie etwa Natriumdioctylsulfosuccinat umfasst.

15. Pharmazeutische Zusammensetzung zur schnellen Freisetzung gemäß Anspruch 12, wobei die Filmbeschichtung ein wasserlösliches Polymer wie etwa Polyvinylpyrrolidon, Polyethylenglykol und Polyvinylalkohol umfasst.

16. Pharmazeutische Zusammensetzung zur schnellen Freisetzung gemäß Anspruch 12, wobei die Filmbeschichtung ein wasserlösliches Polymer in Kombination mit einem Tensid wie etwa Natriumdioctylsulfosuccinat oder Tween umfasst.

## Revendications

1. Composition pharmaceutique à libération rapide, comprenant une quantité efficace de Céfuroxime Axétil, en tant que principe actif, un carbonate alcalin, un acide polycarboxylique en tant qu'agent tampon, et au moins un excipient, dans laquelle ladite composition se présente sous forme de comprimé et comprend de 20 % à 90 % en poids dudit Céfuroxime Axétil, et de 0,1 % à 10 % en poids dudit carbonate alcalin.

2. Composition pharmaceutique à libération rapide selon la revendication 1, comprenant en outre un délitant.

3. Composition pharmaceutique à libération rapide selon la revendication 2, dans laquelle ledit délitant est choisi parmi le groupe comprenant la polyvinylpyrrolidone, la carboxyméthylcellulose sodique, et le glycolate d'amidon sodique.

4. Composition pharmaceutique à libération rapide selon la revendication 2, dans laquelle ledit délitant est insoluble dans l'eau comme la Crospovidone.

5. Composition pharmaceutique à libération rapide selon la revendication 2, comprenant en outre un alcool de sucre en tant que matière de charge, comme le mannitol ou le sorbitol.

6. Composition pharmaceutique à libération rapide selon la revendication 1, dans laquelle ledit carbonate alcalin est choisi parmi le groupe comprenant le bicarbonate de sodium et le bicarbonate de potassium.

7. Composition pharmaceutique à libération rapide selon la revendication 1, dans laquelle ledit acide polycarboxylique est choisi parmi le groupe d'acide citrique ou d'acide tartrique.

8. Composition pharmaceutique à libération rapide selon l'une quelconque des revendications précédentes, dans laquelle ledit au moins un excipient est choisi parmi le groupe comprenant les surfactants et les lubrifiants.

9. Composition pharmaceutique à libération rapide selon la revendication 7, dans laquelle ledit surfactant est choisi parmi le groupe comprenant le lauryl sulfate de sodium et le docusate sodique.

10. Composition pharmaceutique à libération rapide selon l'une quelconque des revendications précédentes, comprenant de 20 % à 90 % en poids dudit Céfuroxime Axétil, de 0,1 % à 10 % en poids dudit carbonate alcalin, de 5 % à 40 % en poids dudit alcool de sucre, de 3 % à 50 % en poids dudit délitant, de 1 % à 30 % en poids dudit acide polycarboxylique et de 1 % à 10 % en poids dudit surfactant.

11. Composition pharmaceutique à libération rapide selon l'une quelconque des revendications précédentes, comprenant de 55 % à 75 % en poids dudit Céfuroxime Axétil, de 8 % à 15 % en poids dudit alcool de sucre, de 10 % à 20 % en poids dudit délitant, de 1 % à 5 % en poids dudit carbonate alcalin et de 1 % à 5 % en poids dudit surfactant.

12. Composition pharmaceutique à libération rapide selon l'une quelconque des revendications précédentes, dans laquelle ladite composition se présente sous forme de comprimé, et qui comprend en outre un pelliculage dont le but est d'améliorer la pénétration de l'eau et de masquer le goût amer dudit Céfuroxime Axétil.

13. Composition pharmaceutique à libération rapide selon la revendication 12, dans laquelle ledit pelliculage représente de 1 % à 5 % en poids de la composition pharmaceutique totale.

14. Composition pharmaceutique à libération rapide selon la revendication 12, dans laquelle ledit pelliculage comprend de 20 % à 99 % en poids d'un polymère soluble comme le PVA, et le PEG, et de 1 % à 20 % en poids d'un surfactant comme le docusate sodique.

15. Composition pharmaceutique à libération rapide selon la revendication 12, dans laquelle ledit pelliculage comprend un polymère soluble dans l'eau comme la polyvinylpyrrolidone, le polyéthylène glycol, et l'alcool polyvinylique.

16. Composition pharmaceutique à libération rapide selon la revendication 12, dans laquelle ledit pelliculage comprend un polymère soluble dans l'eau en combinaison avec un surfactant comme le docusate sodique, ou le Tween.
